# EUROPEAN PATENT APPLICATION

(11) **EP 0 748 793 A1**
(43) Date of publication of application: **18.12.1996**
(21) Application number: 95912527.9
(22) Date of filing: 24.02.1995
(51) Int. Cl.: C07C 235/80, A61K 31/16

(54) **KETOPANTHENOL WITH ACETYLATING PROPERTIES**

(30) Priority: 02.03.1994 RU 94008015
(71) Applicant: TOVARISCHESTVO S OGRANICHENNOI OTVETSTVENNOSTJU " PANT", Moscow 117820 (RU)
(72) Inventor: KOZLOVA, Galina Sergeevna, Moscow, 117513 (RU); SEMINA, Olga Ivanovna, Moscow, 117485 (RU); GUNAR, Vladimir Ivanovich, Moscow, 117393 (RU); KRYLOV, Jury Fedorovich, Moscow, 123007 (RU)
(74) Representative: Desrousseaux, Grégoire Marie
(86) International application number: RU9500033
(87) International publication number: WO9523783

(57) **Abstract**

A novel compound, namely ketopanthenol has been synthesized. It has the following structure:

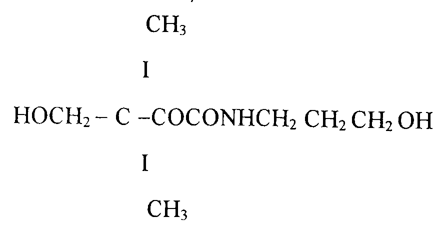

The compound is obtained from the reaction between ketopantolactone and 3-aminopropanol. It has been shown that ketopanthenol has a high level of vitamin activity and can be used in medicine, livestock breeding and cosmetics.

## Description

### Field of invention

The invention concerns the organic chemistry and a novel derivative of pantothenic acid - N-(hydroxy-3,3- dimethyl-3-oxo-1-butyryl)-β-aminopropanol (ketopanthenol), with the structure 1, 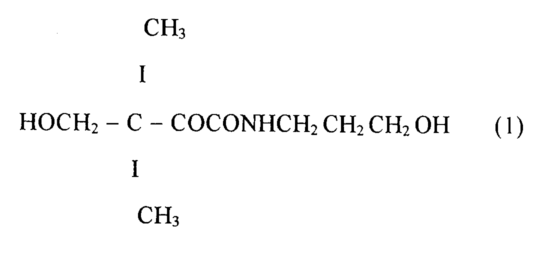 demonstrating acetylating properties, and which can be used in medicine, cosmetics and livestock breeding.

### Background of the invention

At present, the pantothenic acid (vitamin B₃ or B₅) is produced in the form of D,L- and D(+)calcium pantothenate. Only D-pantothenic acid and its salts are biologically active. For medicine and the food industry calcium D-pantothenate is produced. The D,L-form of the agent contains less than 50% of the active substance and is produced for vitaminization of feeds in livestock breeding, The world market price of the vitamin in the D-form is three times higher than the price of the D,L-form.

D-pantothenic acid in the organism is incorporated into the structure of Acetyl coenzyme A (Acetyl CoA), which plays the central role in the metabolism of men and animals. Being an acetylating coenzyme, coenzyme A (CoA), in the acetyl form takes part in the biosynthesis of carbohydrates, fatty acids, amino acids and proteins, stearines, in the synthesis of acetylcholine. Acetyl CoA is necessary for acetylating reactions in the process of biotransformation of alien toxic compounds in the organism in order to make them harmless and to eliminate them from the organism. Reaction with acetic acid is the main way of inactivation of a large number of medical compounds: sulfanilamides, hydrazides, p-aminobenzoic acid derivatives, etc. Acetyl CoA is formed in the organism with the participation of cysteine, adenosine triphosphate and pantothenic acid, the pantothenic acid being the only irreplaceable component in CoA biosynthesis.

The compound which is more close to the claimed compound for its structure is analogue of D-pantothenic acid, namely D-pantothenyl alcohol (D-panthenol), widely used in the world in medicine and cosmetics, which is transformed into pantothenic acid in the cell and demonstrates D-pantothenic acid activity in the organism of people and animals, but has advantages in that it can be applied topically because if its low toxicity and rapid and profound penetration through the skin (Martindale, The Extra Pharmacopoeia, 27th, Ed, p. 1682-3, 1977).

In the synthesis of D-panthenol, D-pantolactone is used, which is obtained in the world by the practice of different methods from synthetic D, L-pantolactone. All the known methods of obtaining D-pantolactone are multistage processes with high usage of the raw materials, solvents, energy and produce a large quantity of waste materials. This process is the most expensive and determines the cost of the vitamin production.

Therefore, the synthesis of novel biologically active compounds with high acetylating properties and lower production cost is of significant importance.

### Summary of the invention

The authors synthesized a novel compound (ketopanthenol) with acetylating activity, analogous to calcium D-pantothenate activity. The distinctive feature of the novel compound is the presence of keto(oxo)pantoic acid and 3-aminopropanol in the structure, forming an amide bond between them. Ketopanthenol is synthesized by the condensation of ketopantolactone and 3-aminopropanol in low aliphatic alcohols as solvent, or without solvent. Thus, the multistage process of separation into enantiomers or ketopantolactone, which is the intermediate of ketopanthenol, is excluded from the ketopanthenol production, which considerably reduces the production cost in comparison with calcium D-pantothenate and D-panthenol.

Ketopanthenol has the following structure (1)

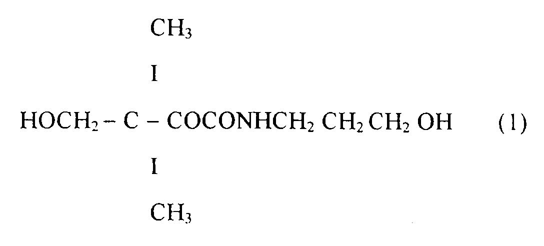

The ketopanthenol structure is proven by the elemental analysis and infra-red spectrum data.

Ketopanthenol is a thick viscous liquid characterized by good solubility in water, alcohols, acetone, chloroform and poor solubility in ether.

### Examples of execution of the invention

### Synthesis of ketopanthenol

### Example 1

3.77 g (0.05 g-mol) of 3-aminopropanol are added to 6.4 g (0.05 g-mol) of ketopantolactone in 15 ml of methanol. The mixture is heated to 60-65° C and is mixed for 2 hours. Then, methanol is distilled off the residue is dissolved in 20 ml of distilled water and mixed with cationic exchanger EO-2 to pH 5.5-6.5. Then the resin is filtered, the filtrate is treated with activated carbon, evaporated in vacuum and dried. 9.85 g of ketopanthenol are obtained.

The output is 97 %; n_{d}²⁰ 1.4930.

| | | | | |
|---|---|---|---|---|
| Found % : | C 52.87 %, | H 8.61 %, | N 6.72 %, | C₉H₁₇O₄N. |
| Calculated for %: | C 53.18 %, | H 8.43 %, | N 6.89 %. | |

Infra-red spectrum: 3350 cm⁻¹ (ν OH, NH), 1700 cm⁻¹(ν C=0),

1660 cm⁻¹(ν CO, amide I), 1520 cm⁻¹(ν CN, amide II).

### Example 2

3.75 g (0.05 g-mol) of 3-aminopropanol are added to 6.4 g (0.05 g-mol) of ketopantolactone. The mixture is heated while being mixed at a temperature of 60-65° C for 2 hours. Then, it is dissolved in distilled water, filtered through activated carbon, evaporated in vacuum and dried. 9.91 g of ketopanthenol are obtained.

Output is 97 % ; n_{d}²⁰ 1.4925.

### Example 3

3.75 g (0.05 g-mol) of 3-aminopropanol are added to 6.4 g (0.05 g-mol) of ketopantolactone. The mixture is heated to 60-65° C and mixed for 2 hours. Then, 20 ml of methanol are added to the mixture and mixed till it is fully dissolved. The resulting solution is cooled and filtered through activated carbon. Methanol is distilled off, the residue is dried in vacuum. 9.78 g of ketopanthenol are obtained.

Output is 96.2 %.

### Examination of Acetylating properties

The level of the organism supply with a vitamin is determined by the study of the reaction of acetylation of exogenous aromatic amines. The total acetylating properties of the organism were assessed by the method of quantitative determination of the content of arylamines in the examined substrate (urine).

### Example 4

Sodium sulfacyl was chosen as the substance for acetylation.

The influence of 4-days of injections of ketopanthenol on the acetylating properties of white rats was examined during the experiments.

The experiments were carried out using mongrel pubescent male rats. 14 animals were included in each group. The examined agent was injected intraperitoneally in a dose of 29 mg/kg. On the 4th day 4 hours after the last injection of the examined agent the sodium sulfacyl solution in a dose of 20 mg/kg was injected intraperitoneally. All the rats were separated into exchangeable cages for urine gathering. The resulting data were compared with the control groups (group I - only received sodium sulfacyl in a dose of 20 mg/kg, and group II received calcium D-pantothenate).

The average weight of the animals in the group fluctuated within 154.7 ± 10.8 g, the absolute dose of sodium sulfacyl was 3.09 ± 0.14 mg.

The changes of diuresis showed that under the influence of ketopanthenol the diuresis of experimental animals tended to some lower values in comparison with the control group.

Analysis of the results of the experiments shows that ketopanthenol increased the excretion of the acetylated product 1.7 times in comparison with group II and 4 times in comparison with the control group, along with it the excretion of the sulfacyl in its free form was 20 % lower when compared with the series I (Tables 1 and 2).

The acetylating properties of the organism influenced by ketopanthenol increased 1.5 times in comparison with calcium D-pantothenate and 3.6 times in comparison with the control.

Therefore, the results of the experiments showed that the total acetylating capability of the white rat, according to the test of acetylation of aromatic amines (N-acetylation) increased significantly under the influence of ketopanthenol. Along with it the ketopanthenol activity was 1.5 times higher than calcium D-pantothenate activity.

### Examination of toxicity

### Example 5

To determine acute toxicity of ketopanthenol, non-linear white mice of both sexes with the average mass of 19 ± 3 g were used. There were 72 mice in the experiment, the animals were divided into 12 groups, 6 animals in each group. The mice of the first 6 groups received ketopanthenol in 0.2 ml of distilled water once internally in doses: 20.0, 21.0, 22.0, 23.0, 24.0 and 25.0 g/kg. The next 6 groups in the same conditions received D-panthenol for comparison in doses: 20.0, 21.0, 22.0, 22.5, 23.0, 23.5 g/kg of mass.

The 12 group was the control one, the mice of this group received once internally only 0.2 ml of distilled water.

The mice were under observation for 14 days.

The mice which received ketopanthenol, starting from the dose of 22.0 g/kg immediately after the injection lost mobility, had quickened breathing, inertia, narrowed eye slots. The animals started to die 1-2 days after the injection. The maximum endurable dose of ketopanthenol was shown to be the dose of 21.0 g/kg of mass, analogous to D-panthenol.

The LD₅₀ calculations were carried out according to Kerber

For ketopanthenol LD₅₀ - 23.5 g/kg, LD₁₀₀ - 25.0 g/kg.

For D - panthenol LD₅₀ - 22.2 g/kg, LD₁₀₀ - 23.5 g/kg.

Therefore, based on the results of the experiments the conclusion was made that ketopanthenol is a low toxic compound.

### Example 6

The study of subchronic toxicity and the local irritating effect of ketopanthenol.

In the experiment carried out for 2 months, non-linear white rats of both sexes with the average initial weight of 75 ± 3.3 g were used. The animals were divided into 7 groups. The first three groups received daily, during the whole experimental period, ketopanthenol in 0.2 ml of distilled water in doses of 13.2 mg/kg (which corresponds to the therapeutic dose of vitamin B₃₎, 132.0 and 264 mg/kg (in doses 10 and 20 times larger than the therapeutic dose). Then three groups of rats under experiment received D-panthenol in the same conditions and doses (for comparison). The animals of the seventh group were used as control, receiving internally 0.2 ml of distilled water daily.

During the whole experiment the animals were weighed once a week and the observation on their development, state and behavior was carried out.

No changes in development, state and behavior of the animals receiving ketopanthenol in comparison with the control rats were observed.

The growth of the mass of rats receiving the examined agents during the experiment, is presented in table 3, which shows that ketopanthenol being injected duiing a prolonged period has no toxic influence on the animals' organism and demonstrates vitamin activity. The growth of the mass of the rats which received ketopanthenol in therapeutic dose and in doses 10 and 20 times larger than the therapeutic dose, was 10.7, 12.0 and 6.5 % higher correspondingly in comparison with the control animals. The growth of mass of animals receiving D-panthenol was 8.0, 9.3 and 5.2 % correspondingly in comparison with the control.

When the experiment was over after the slaughtering and visual examination of the internal organs no local irritating effect was observed.

The aqueous solution of ketopanthenol was dripped into the conjunctive bag of the eye of 5 rabbits weighing 2.5 ± 0.3 kg for three days. No irritating effect was observed.

Thus, the study of subchronic toxicity and irritating effect of ketopanthenol showed that the examined agent has no toxic effect, being used during a prolonged period and its biologic activity is slightly higher than D - panthenol activity.

### Industrial applicability.

The advantages of the declared compound in comparison with the well-known D-panthenol and calcium D-pantothenate are the enhanced acetylating capabilities and considerably lower production cost.

The results of the experiments demonstrate that the new compound has vitamin activity and that it could be used in medicine, cosmetics, livestock breeding, both directly and as a component of other agents, such as a vitamin additive.

**Table 1**

| N | Group of animals, Agent | Sulfacyl dose, M± m | Diuresis M± m |
|---|---|---|---|
| 1 | Group I control (Na sulfacyl) | 3.13 ± 0.09 | 3.4 ± 0.18 |
| 2 | Group II (D (+) pantothenate Ca & Na sulfacyl) | 3.0 ± 0.08 | 3.9 ± 0.2 |
| 3 | Group III (ketopanthenol Na sulfacyl) | 3.09 ± 0.14 | 1.7 ± 0.3 |

Continuation of table 1

| N | Na sulfacyl excretion | | | |
|---|---|---|---|---|
| | Sulfacyl isolation M ± m | | | Excretion % from the dose |
| | In free form | In acetylated form | Total | |
| 1 | 2.34 ± 0.13 | 0.28 ± 0.03 | 2.62 ± 0.17 | 83.9 |
| 2 | 2.12 ± 0.09 | 0.67 ± 0.04 | 2.79 ± 0.19 | 93.0 |
| 3 | 1.92 ± 0.09 | 0.99 ± 0.4 | 2.91 ± 0.21 | 94.2 |

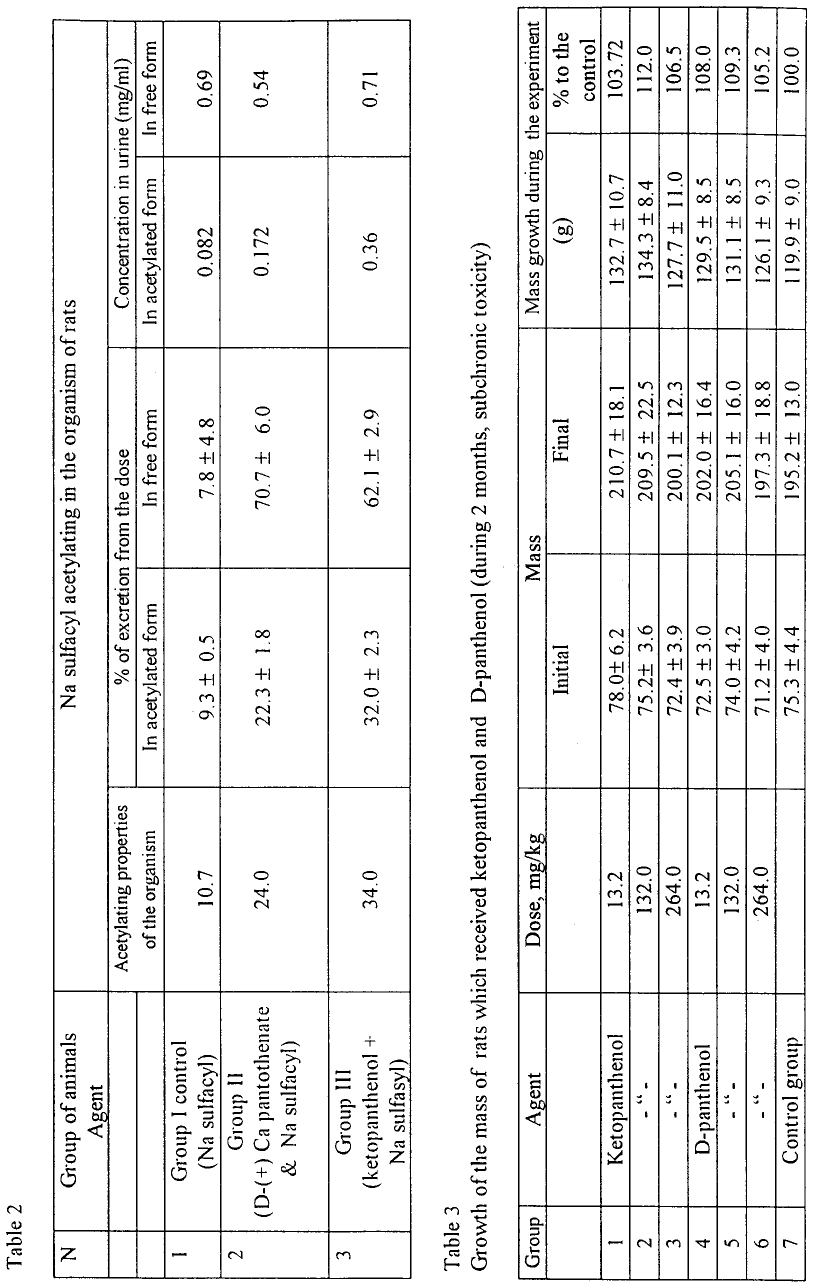

## Claims

1. Ketopanthenol, with the structure (1)

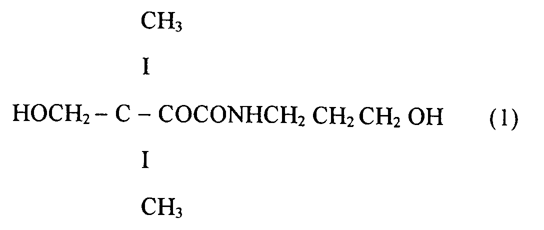

2. The method of obtaining of ketopanthenol with structure (1) is characterized by condensation of ketopantolactone with 3-aminopropanol.

3. The method of clause 2 which is distinguished by the fact that the process is carried out in low aliphatic alcohols as solvent.

4. Ketopanthenol with structure (1) demonstrating acetylating properties in the bodies of people and animals.

5. Usage of ketopanthenol with structure (1) as a vitamin agent.

6. Usage of ketopanthenol with structure (1) for the production of vitamin remedies.

7. Usage of ketopanthenol with structure (1) as a vitamin additive to cosmetic products.
